# EUROPÄISCHE PATENTSCHRIFT

(11) **EP 0 512 980 B1**
(45) Veröffentlichungstag und Bekanntmachung des Hinweises auf die Patenterteilung: **07.12.1994**
(21) Anmeldenummer: 89903714.7
(22) Anmeldetag: 21.03.1989
(51) Int. Cl.: A61N 5/08

(54) **MEDIZINISCHES UV-BESTRAHLUNGSGERÄT**
MEDICINAL U/V IRRADIATION DEVICE
APPAREIL MEDICAL D'IRRADIATION AUX RAYONS UV

(43) Veröffentlichungstag der Anmeldung: 19.11.1992
(73) Patentinhaber: Mutzhas, Irmgard, D-81479 München (DE)
(72) Erfinder: Mutzhas, Maximilian F., Prof. Dr., - (DE)
(74) Vertreter: Viering, Jentschura & Partner
(86) Internationale Anmeldenummer: EP8900308
(87) Internationale Veröffentlichungsnummer: WO9011105

(56) Entgegenhaltungen:
- EP-A- 0 023 311
- EP-A- 0 275 817
- WO-A-87/02256

## Beschreibung

Die Erfindung berifft ein medizinisches UV-Bestrahlungsgerät, zur Verbesserung des Immunsystems und/oder zur Bildung krebshemmender Stoffe.

Ein erheblicher Prozentsatz der Bevölkerung leidet an Infektions-, Krebs- und Immunerkrankungen sowie an Krankheiten, die im wesentlichen darauf zurückzuführen sind, daß das Immunsystem zu wenig wirksam bzw. der Pegel an krebshemmenden Stoffen zu niedrig ist. Als vorbeugende Maßnahmen gegen die oben erwähnten Erkrankungen werden im wesentlichen auf physikalischer Basis beruhende Bewegungs-, Wasser-, Bäder- und ähnliche Therapien angewandt. Anßerdem finden gezielte Vitamin- und Mineralstoffgaben sowie Sauerstofftherapien Anwendung. Bei all diesen therapeutischen Verfahren ist der Nachweis ihrer Wirksamkeit äußerst schwierig, ihre medizinische Wirksamkeit somit umstritten. Therapeutische Verfahren auf der Basis von Schutzimpfungen geben bisher nur einen wirksamen Schutz gegen ganz spezifische Erkrankungen, nicht jedoch eine breitbandige Immunverbesserung, die die körpereigene Abwehr gegen eine Vielzahl von möglichen Erkrankungen in ihrer Wirkung steigert bzw. zur Krebshemmung beiträgt.

Aus der EP-A-0 023 311 ist ein medizinisches Bestrahlungsgerät bekannt, bei dem Frequenzen von weniger als 350 nm ausgefiltert sind, um die Bestrahlungsdauer nicht durch Erythembildung zu begrenzen. Das Maximum der Emission soll bei etwa 370 nm liegen. Durch die Bestrahlung soll eine Regeneration von krankhaft verändertem Zellgewebe erreicht werden. Die dazu erforderlichen Bestrahlungsstärken sind nicht spezifiziert.

Nach der WO 87/02256 wird UV-Licht zur Bestrahlung von Wunden eingesetzt, wobei der UV-B-Anteil im Bereich zwischen 280 und 315 nm ausgefiltert wird. Mittels einer Fokussiereinrichtung wird das Licht einer Quecksilber-Hochdrucklampe auf eine Fläche von 25 bis etwa 200 mm² fokussiert.Die Bestrahlungsstärke im UV-C-Bereich (280-315 nm) beträgt auf einer Fläche von 50 cm² etwa 10 bis 20 W/m² und im UV-A-Bereich (315-400 nm) etwa 20-40 W/m².

Der Erfindung liegt die Aufgabe zugrunde, eine medizinische Behandlungseinrichtung zu entwickeln, die eine Verbesserung des Immunsystems und/oder die Bildung krebshemmender Stoffe ermöglicht.

Diese Aufgabe wird erfindungsgemäß durch ein zur Verbesserung des Immunsystems und/oder zur Bildung krebshemmender Stoffe bestimmtes medizinisches UV-Bestrahlungsgerät gelöst, dessen Spektralanteil zwischen 250 und 350 nm weniger als 2%, vorzugsweise weniger als 1%, vorzugsweise weniger als 0,2%, der gesamten, zwischen 250 und 400 nm abgestrahlten UV-Strahlung beträgt und dessen in der Nutzebene vorhandene Bestrahlungsstärke im Wellenlängenbereich zwischen 350 und 400 nm zwischen 400 und 2000 W·m², vorzugsweise zwischen 600 und 2000 W·m² beträgt.

Sowohl in Bezug auf die Hautkrebsentstehung, als auch hinsichtlich der Immunreaktion herrschen bisher in der Fachwelt völlig widersprüchliche Meinungen. UV-A-Bestrahlungsversuche mit einem Kollektiv gesunder Versuchspersonen zeigten, daß einige für das Immunsystem wichtige Parameter zum Negativen hin verändert wurden. Versuche, in denen haarlose Mäuse mit Strahlungsquellen verschiedener spektraler Energieverteilungen bestrahlt wurden, zeigten, daß UV-A-Strahlung eine geringe Verbesserung der Immunreaktion auslöste. Einige Versuche mit haarlosen Mäusen zeigten, daß UV-A und UV-B in Bezug auf das Risiko der Hautkrebsentstehung in der gleichen Größenordnung liegen. Ein weiterer Versuch mit UV-A-Bestrahlung von haarlosen Mäusen zeigte, daß keine Hautkarzinome ausgelöst werden können, wogegen andere Experimente mit haarlosen Mäusen ergaben, daß UV-A die karzinogene Wirkung des UV-B verstärkt. Weitere Experimente wiederum zeigten, daß die Vorbestrahlung bzw. Nachbestrahlung mit UV-A das Karzinomrisiko mindern kann. Diese bisher in der Fachwelt bestehenden Widersprüche ließen sich nun durch die der vorliegenden Erfindung zugrundeliegenden, aufwendigen photobiologischen Analysen und eingehenden Experimente aufklären. Dabei stellte sich überraschend heraus, daß der Bereich der UV-Strahlung unterhalb von 350 nm (d.h. der Bereich zwischen 250 wund 350 nm) negative Auswirkungen auf das Immunsystem hat, während das langwellige UV-A, dessen Bereich oberhalb von 350 nm liegt, positive Auswirkungen auf das Immunsystem zeigt bzw. krebshemmende Stoffe bildet.

Neben dieser positiven Wirkung sind auch die möglichen Nebenwirkungen in Betracht zu ziehen. Dabei geht es im wesentlichen um akute und chronische Wirkungen auf die Haut, da diese als Empfängerorgan verwendet wird.

Akute Nebenwirkungen sind Erythem sowie phototoxische und photoallergische Reaktionen. Chronische Nebenwirkungen sind Photokarzinogenese sowie aktinische Elastose. Im Bereich oberhalb von 350 nm haben die vorher erwähnten Nebenwirkungen ein Minimum. Die einzige klinisch feststellbare Nebenwirkung bei der Bestrahlung in Wellenlängenbereich oberhalb von 350 nm ist eine Hautpigmentierung, die jedoch von Patienten als positiv bewertet wird. Der Erfindung liegt damit die Erkenntnis zugrunde, daß für die Verbesserung des Immunsystems bzw. für die Bildung krebshemmender Stoffe die Ultraviolett-Strahlung im Wellenlängenbereich zwischen 350 und 400 nm am günstigsten ist: Diese Strahlung kann lokal und/oder systemisch wirken. Die dabei gebildete wirksame immunpositive, krebshemmende biologische Materie kann sowohl zu aktiver als auch zu passiver Immunisierug bzw. Krebshemmung benutzt werden. Der unterhalb von 350 nm liegende Strahlungsanteil führt demgegenüber nicht nur zu unerwünschten Nebenwirkungen, sondern vermindert gleichzeitig die immunpositive bzw. krebshemmende Wirkung der Strahlung oberhalb von 350 nm. Er kann somit zu einer Umkehrung des gewünschten therapeutischen Effektes führen.

Die immunpositive bzw. krebshemmende Wirksamkeit des Bestrahlungsgerätes hängt im wesentlichen von der verabreichten Strahlungsdosis ab. Diese wiederum läßt sich als Produkt aus Bestrahlungsstärke mal Bestrahlungszeit dann günstiger applizieren, wenn die Bestrahlungsstärke in der Nutzebene entsprechende Werte annimmt. Unterhalb und oberhalb bestimmter Grenzwerte läßt sich keine vernünftige photobiologische Wirkung erzielen. Ist die Untergrenze zu niedrig, dauert es zu lange, bis die erwünschte Wirkung eintritt. Ist die Obergrenze zu hoch, kann es durch Überhitzung zu Störungen im biologischen System kommen. Bei der Bestrahlung von Menschen liegt die Schmerzgrenze bei der Hauttemperatur von ca. 41 °C. Würde diese Temperatur durch zu hohe Bestrahlungsstärke überschritten, sind geeignete Kühlungsmaßnahmen notwendig, wie Luftkühlung durch Gebläse oder Wasserkühlung. Letztere kann durch Besprühen oder durch Unterwasserbehandlung erfolgen, wobei in diesen Fällen durch die Wassersättigung des Stratum Corneum eine verbesserte Transmission der Epidermis erzielt wird.

Die Bestrahlungsstärke in der Nutzebene im Wellenlängenbereich zwischeen 350 und 400 nm liegt deshalb zwischen 400 und 2000 W· m⁻², vorzugsweise zwischen 600 und 2000 W·m⁻².

Die an das Ultraviolett angrenzende sichtbare Strahlung im Bereich des Violettem zwischen 400 und 440 nm trägt ebenfalls zu dieser immunpositiven bzw. krebshemmenden Wirkung bei. Ihr Anteil in der Nutzebene soll deshalb im Wellenlängenbereich zwischen 400 und 440 nm höchstens 200%, vorzugsweise höchstens 50%, vorzugsweise höchstens 20% der Bestrahlungsstärke zwischen 350 und 400 nm betragen. Wird die Bestrahlungsstärke im Bereich zwischen 400 und 440 nm zu hoch, so entsteht die nachteilige Wirkung, daß die strahlungsbedingte Energiebelastung für den Organismus nicht mehr tolerierbar ist.

Strahlung oberhalb von 440 nm trägt nicht mehr signifikant zu dieser immunpositiven bzw. krebshemenden Wirkung bei, deshalb soll sie begrenzt werden. So soll die in der Nutzebene vorhandene Bestrahlungsstärke im Wellenlängenbereich zwischen 440 und 800 nm höchstens 150%, vorzugsweise höchstens 75%, vorzugsweise höchstens 50%, weiterhin vorzugsweise höchstens 5% der Bestrahlungsstärke im Wellenlängenbereich zwischen 350 und 400 nm betragen.

Strahlung oberhalb von 800 nm führt in entsprechend hoher Dosierung zu Hautkarzinomen und zu aktinischer Elastose. Sie trägt zur immunpositioen bzw. krebshemmenden Wirkung nicht bei, deshalb soll im speziellen das kurz- und mittelwellige Infrarot begrenzt werden, so daß die in der Nutzebene vorhandene Bestrahlungsstärke im Wellenlängenbereich von 800 bis 3000 nm höchstens 50%, vorzugsweise höchstens 20%, vorzugsweise höchstens 10%, weiterhin vorzugsweise höchstens 5% der Bestrahlungsstärke zwischen 350 und 400 nm beträgt.

Zur weiteren Erläuteruung der Erfindung dienen folgende Beispiele mit am Prioritätstage auf dem Markt erhältlichen Strahlungsquellen, Reflektoren, Filtern und Filtersubstanzen.

### Beispiel 1:

Strahlungsquelle: 10 Metallhalogendampfstrahler à 400 W, dessen wesentliches Halogenid Eisenjodid ist.
Filter: Polycarbonat 2 mm Dicke mit 2% Filtersubstanz UVASUN 105.
Bestrahlungsstärke:

| | |
|---|---|
| 250 - 350 nm: | 4,1 W·m⁻² |
| 350 - 400 nm: | 400 W·m⁻² |
| 400 - 440 nm: | 168 W·m⁻² |
| 440 - 800 nm: | < 20 W·m⁻² |
| 800 - 3000 nm: | < 20 W·m⁻² |

### Beispiel 2:

Strahlungsquelle: 3 Metallhalogendampfstrahler à 4000 W, dessen wesentliches Halogenid Eisenjodid ist.
Filter: Gehärtete Glasplatte 5 mm Dicke, sowie Schott-Glasfilter UG 1,2 mm, überzogen mit 0,1 mm Acryllackschicht, die 8 % Filtersubstanz UVASUN 104 enthält.
Bestrahlungsstärke:

| | |
|---|---|
| 250 - 350 nm: | 4,2 W·m⁻² |
| 350 - 400 nm: | 600 W·m⁻² |
| 400 - 440 nm: | 90 W·m⁻² |
| 440 - 800 nm: | < 100 W·m⁻² |
| 800 - 3000 nm: | < 100 W·m⁻² |

### Beispiel 3:

Strahlungsquelle: 8 Metallhalogendampfstrahler à 4000 W, dessen wesentliches Halogenid Eisenjodid ist.
Filter: Acrylglasplatte 6 mm Dicke mit 1,6% Filtersubstanz UVASUN 112 sowie Schott-Glasfilter UG 1,2 mm Dicke und Schott-Glasfilter KG 1,6 mm Dicke.
Bestrahlungsstärke:

| | |
|---|---|
| 250 - 350 nm: | 1 W·m⁻² |
| 350 - 400 nm: | 1200 W·m⁻² |
| 400 - 440 nm: | 180 W·m⁻² |
| 440 - 800 nm: | < 60 W·m⁻² |
| 800 - 3000 nm: | < 60 W·m⁻² |

In den Beispielen 1 bis 3 enthält das Bestrahlungsgerät eloxierte Aluminium-Reflektoren sowie ein Luftkühlungssystem, um Strahler, Filter und Reflektoren auf der optimalen Temperatur zu halten.

Die ausgewiesenen UVASUN Filtersubstanzen und Farbstoffe sind Produkte der Mutzhas Produktions-GmbH München.

In den Beispielen 1 bis 3 beziehen sich die Bestrahlungsstärkewerte auf die Nutzfläche gemäß DIN 5050. Sämtliche Beispiele beziehen sich auf Ganzkörper-Bestrahlungsgeräte, da hierbei die systemischen Effekte höher sind als bei Teilkörper-Bestrahlungsgeräten.

Durch Erhöhung der Konzentration der Filtersubstanzen in den entsprechenden Substraten kann der Anteil der Strahlung unterhalb von 350 nm praktisch vollständig eliminiert werden, dies geht jedoch wegen der endlichen Kantensteilheit mit einer Verminderung der Bestrahlungsstärke im Bereich von 350 bis 400 nm einher.

Zur weiteren Erläuterung des mit dem erfindungsgemäßen UV-Bestrahlungsgerät erzielten therapeutischen Effektes sei folgendes Behandlungsbeispiel angeführt:

Es wurde eine Ganzkörper-Phototherapie mit erfindungsgemäßen Bestrahlungsgeräten durchgeführt, wobei fünf bis zwanzig Bestrahlungen innerhalb von zwei bis drei Wochen mit jeweils 15 bis 60 min. Bestrahlungszeit dunchgeführt wurden. Die Bestrahlungsstärke betrug 400 bis 1200 W·m⁻² im Bereich von 350 bis 400 nm, wobei die im Bereich von 350 bis 400 nm verabreichte Einzeldosis zwischen 360 000 und 4 320 000 J·m⁻² betrug und die Gesamtdosis der Therapieserie zwischen 1 800 000 und 86 400 000 J·m⁻² lag.

Nach dieser Phototherapie zeigte sich eine Zunahne der Zahl der T-Helfer-Lymphozyten, der Monozyten (Makrophagen), der Granulozyten sowie des T-Helfer/T-Suppressor-Quotienten um über 20%. Gleichzeitig konnte eine Steigerung der Lymphozyten-Aktivität und der Hautreaktionen vom Spättyp auf Recall-Antigene um etwa 100% beobachtet werden.

Im Falle der passiven Immunisierung bzw. Krebshemmung lassen sich entweder durch die Bestrahlung von Menschen oder durch die Bestrahlung von Tieren die in diesen entstehende biologisch wirksamen Stoffe gewinnen. Diese können, gegebenenfalls entsprechend konzentriert, durch Injektionen, orale oder topische Gaben appliziert werden. Die immunpositiven bzw. krebshemmenden Stoffe können aus Seren (Blut, Lymphe etc.) bzw. aus Zellen gewonnen werden. Im Falle der Bestrahlung von Menschen und Tieren zur Gewinnung dieser immunpositiven bzw. krebshemmenden Stoffe haben sich Ganzkörperbestrahlungen als besonders wirksam erwiesen.

Werden diese immunpositiven bzw. krebshemmenden Stoffe aus Tieren gewonnen, so ist die Wirksamkeit deutlich erhöht, wenn deren Haut kein Feder- oder Haarkleid trägt, da beide das Eindringen der wirksamen Strahlung in die lebende Materie verhindern. Geeignet für diese Zwecke sind haarlose Züchtungen, wie sie bei Mäusen bereits zur Verfügung stehen und wie sie über genetische Manipulation auch bei anderen Wirbeltieren gezüchtet werden können.

Durch extrakorporale Blutbestrahlung sowie durch in vitro Bestrahlung (z.B. von Zellkulturen) lassen sich die immunpositiven bzw. krebshemmenden Stoffe ebenfalls gewinnen.

Das Bestrahlungsgerät zur Bildung immunpositiver bzw. krebshemmender Stoffe kann im Bereich der kosmetischen Bräunung eingesetzt werden, sowohl im gewerblichen als auch im privaten Bereich. Bei manchen beruflichen Tätigkeiten ist der Umgang mit Immunsuppressiva bzw. Karzinogenen unvermeidbar. Selbst geringe Mengen bzw. Konzentrationen dieser photobiologischen, chemischen oder physikalischen Immunsuppressiva bzw. Karzinogene können immunsuppressiv bzw. krebsauslösend wirken. Dieses Risiko kann durch vorbeugende Bestrahlungskuren oder durch nachträgliche Bestrahlungskuren vermindert werden.

## Patentansprüche

1. Medizinisches UV-Bestrahlungsgerät zur Verbesserung des Immunsystems und/oder zur Bildung krebshemmender Stoffe, mit folgenden Merkmalen:
a) Der Spektralanteil zwischen 250 und 350 nm beträgt weniger als 2%, vorzugsweise weniger als 1%, vorzugsweise weniger als 0,2% der gesamten zwischen 250 und 400 nm abgestrahlten UV-Strahlung
b) die in der Nutzebene vorhandene Bestrahlungsstärke beträgt im Wellenlängenbereich zwischen 350 und 400 nm zwischen 400 und 2000 W·m⁻², vorzugsweise zwischen 600 und 2000 W·m⁻².

2. Bestrahlungsgerät nach Anspruch 1, dadurch gekennzeichnet, daß die in der Nutzebene vorhandene Bestrahlungsstärke im Wellenlängenbereich zwischen 400 und 440 nm höchstens 200%, vorzugsweise höchstens 50%, vorzugsweise höchstens 20% der Bestrahlungsstärke zwischen 350 und 400 nm beträgt.

3. Bestrahlungsgerät nach Ansprnch 1, dadurch gekennzeichnet, daß die in der Nutzebene vorhandene Bestrahlungsstärke im Wellenlängenbereich zwischen 440 und 800 nm höchstens 150%, vorzugsweise höchstens 75%, vorzugsweise höchstens 10%, vorzugsweise höchstens 5% der Bestrahlungsstärke zwischen 350 und 400 nm beträgt.

4. Bestrahlungsgerät nach Anspruch 1, dadurch gekennzeichnet, daß die in der Nutzebene vorhandene Bestrahlungsstärke im Wellenlängenbereich zwischen 800 und 3000 nm höchstens 50%, vorzugsweise höchstens 20%, vorzugsweise höchstens 10%, vorzugsweise höchstens 5% der Bestrahlungsstärke zwischen 350 und 400 nm beträgt.

## Claims

1. Medicinal UV-irradiation device for the improvement of the immune system and/or for the formation of cancer inhibiting substances, characterized in that
a) the spectral ratio between 250 and 350 nanometers is less than 2 %, preferably less than 1 %, preferably less than 0.2 % of the whole UV-radiation emitted between 250 and 400 nm
b) the intensity of radiation in the target area at a range of wavelengths between 350 and 400 nanometers is between 400 and 2000 W·m⁻², preferably between 600 and 2000 W·m⁻².

2. Irradiation device according to claim 1, characterized in that the intensity of radition in the target area at a range of wavelengths between 400 and 440 nanometers is at the most 200 %, preferably at the most 50 %, preferably at the most 20 % of the intensity of radiation between 350 and 400 nanometers.

3. Irradiation device according to claim 1, characterized in that the intensity of radiation in the target area at a range of wavelengths between 440 and 800 nanometers is at the most 150 %, preferably at the most 75 %, preferably at the most 10 %, preferably at the most 5 % of the intensity of radiation between 350 and 400 nanometers.

4. Irradiation device according to claim 1, characterized in that the intensity of radiation in the target area at a range of wavelengths between 800 and 3000 nanometers is at the most 50 %, preferably at the most 20 %, preferably at the most 10 %, preferably at the most 5 % of the intensity of radiation between 350 and 400 nanometers.

## Revendications

1. Appareil médical d'irradiation aux rayons UV pour l'amélioration de l'immunosystème et/ou pour la formation des substances inhibitifs de cancer, characterisé en ce que
a) le pourcentage du spectre entre 250 et 350 nanomètres est moins que 2 %, de préférence moins que 1 %, de préférence moins que 0.2 % de la totalité de rayons UV émise entre 250 et 400 nanomètres
b) la intensité de radiation à la surface utile dans la région de longueur d'ondes entre 350 et 400 nanomètres est entre 400 et 2000 W·m⁻², de préférence entre 600 et 2000 W·m⁻².

2. Appareil d'irradiation selon la revendication 1, characterisé en ce que la intensité de radiation à la surface utile dans la région de longueur d'ondes entre 400 et 440 nanomètres est au maximum 203 %, de préférence au maximum 50 %, de préférence au maximum 20 % de la intensité de radiation entre 350 et 400 nanomètres.

3. Appareil d'irradiation selon la revendication 1, characterisé en ce que la intensité de radiation à la surface utile dans la région de longueur d'ondes entre 440 et 800 nanomètres est au maximum 75 %, de préférence au maximum 10 %, de préférence au maximum 5 % de la intensité de radiation entre 350 et 400 nanomètres.

4. Appareil d'irradiation selon la revendication 1, characterisé en ce que la intensité de radiation à la surface utile dans la région de longueur d'ondes entre 800 et 3000 nanomètres est au maximum 50 %, de préférence au maximum 20 %, de préférence au maximum 10 %, de préférence au maximum 5 % de la intensité de radiation entre 350 et 400 nanomètres.
